# FASCICULE DE BREVET EUROPEEN

(11) **EP 0 494 022 B1**
(45) Date de publication et mention de la délivrance du brevet: **08.10.1997**
(21) Numéro de dépôt: 91403559.7
(22) Date de dépôt: 27.12.1991
(51) Int. Cl.: C08F 220/54

(54) **Nouveaux copolymères à base de N-alkyl acrylamide, leur préparation et leur utilisation comme agents épaississants, notamment dans des compositions de shampooing**
Copolymere auf Basis von N-Alkylacrylamid, deren Herstellung und deren Verwendung als Verdickungsmittel in Shampoozusammensetzungen
Copolymers based on N-alkyl acrylamide, their preparation and their use as thickeners in shampoo compositions

(30) Priorité: 27.12.1990 FR 9016307
(43) Date de publication de la demande: 08.07.1992
(73) Titulaire: L'OREAL, 75008 Paris (FR)
(72) Inventeur: Mondet, Jean, F-93700 Drancy (FR); Lion, Bertrand, F-93190 Livry-Gargan (FR)
(74) Mandataire: Nony, Michel

(56) Documents cités:
- EP-A- 0 364 887
- FR-A- 2 180 006
- US-A- 4 423 199

## Description

La présente invention a pour objet l'utilisation comme agents épaississants de copolymères de N-alkyl acrylamide.

On sait que les sels de polymères provenant de la polymérisation d'acides organiques carboxyliques insaturés, tels que les sels d'acides polyacryliques et polyméthacryliques, sont utilisés comme agents épaississants dans divers systèmes aqueux.

L'épaississement des solutions de tensioactifs ioniques (par exemple des compositions de shampooing) pose des problèmes particuliers. Les agents épaississants sont très utiles dans de telles compositions auxquelles ils confèrent de l'onctuosité. En outre, l'épaississement de la solution de tensioactifs favorise l'emploi par l'utilisateur, en évitant que des quantités trop importantes de la solution de tensioactifs ne se déverse lorsque l'on incline le flacon contenant la composition. En outre, l'agent épaississant évite ou limite la décantation de certains ingrédients présents dans la composition.

L'un des moyens les plus simples pour augmenter la viscosité des solutions de tensioactifs anioniques est d'ajouter un électrolyte, généralement un sel minéral tel que le chlorure de sodium ou le chlorure d'ammonium.

Cependant, les sels minéraux ne peuvent généralement pas être utilisés comme seuls agents épaississants, car cela nécessiterait des quantités trop importantes, incompatibles avec certaines utilisations.

L'un des inconvénients des polymères dérivés d'acides carboxyliques insaturés est qu'ils sont incompatibles avec les sels minéraux. Par exemple, les systèmes épaissis avec de tels polymères ont une viscosité qui décroît fortement lorsque l'on ajoute un électrolyte tel que le chlorure de sodium ; voir par exemple le brevet US 4,423,199.

Dans ce brevet US 4,423,199, on préconise l'utilisation de polymères d'acides carboxyliques insaturés, modifiés par la présence d'autres comonomères, et en particulier des dérivés de N-alkyl acrylamide à longue chaîne alkyle.

Les seuls copolymères concrètement décrits dans ce brevet US proviennent de mélanges de comonomères contenant 5 % en poids de N-alkyl acrylamide à longue chaîne. La description indique que le mélange de comonomères de départ peut contenir de 0,5 à 25 % en poids de N-alkyl acrylamide, et de préférence de 1 à 15 % en poids.

Les copolymères épaississants décrits dans ce brevet US peuvent être utilisés en présence de tensioactifs, généralement jusqu'à une concentration de 2,5 % de tensioactifs, la teneur utile en tensioactifs étant, selon ce brevet, comprise entre 0,1 et 1,0 % en poids environ.

L'un des inconvénients des copolymères décrits dans le brevet US 4,423,199 est que leur effet épaississant passe par un maximum, puis diminue à mesure que la teneur en tensioactifs augmente.

Les copolymères décrits dans ce brevet US ne peuvent pas convenir pour épaissir des milieux dans lesquels la concentration en tensioactifs est assez élevée. C'est le cas par exemple de certaines compositions cosmétiques telles que des shampooings, pour lesquelles la concentration en tensioactifs est généralement supérieure à 5 % en poids. En effet, avec les copolymères effectivement décrits dans le brevet US 4,423,199 (contenant 5 % de motifs N-alkyl acrylamide), la viscosité est certes augmentée pour des faibles concentrations de tensioactifs, comprises entre 0,1 et 3 % en poids, mais cette viscosité devient à nouveau très faible pour des concentrations supérieures de tensioactif, même en présence d'une concentration en électrolyte minéral minéral supérieure ou égale à 1 % en poids dans la solution.

Même en utilisant des copolymères, tels qu'envisagés par le brevet US 4,423,199, contenant de 20 à 25 % en poids de motifs N-alkyl acrylamide, il est possible d'augmenter la viscosité de certains tensio-actifs en solution, comme le lauryl sulfate de sodium à une concentration de 8 %, mais la texture des gels obtenus est trop filante à l'écoulement et ne peut donc convenir en pratique.

On a maintenant découvert qu'il est possible d'épaissir des milieux contenant un ou plusieurs tensioactifs à une concentration supérieure ou égale à 5 % en poids, grâce à l'utilisation de nouveaux copolymères contenant, outre des motifs dérivés d'acides organiques carboxyliques insaturés, au moins 30 % en poids de motifs dérivés de N-alkyl acrylamide à longue chaîne.

Les copolymères utilisés proviennent de la copolymérisation d'un mélange de monomères comprenant :
- au moins 30 % en poids d'au moins un acide organique carboxylique à insaturation éthylénique ;
- au moins 30 % en poids d'au moins un N-alkyl acrylamide dont le groupement alkyle contient de 8 à 30 atomes de carbone dans la chaîne alkyle ;
- et de 0 à 40 % d'un monomère insaturé hydrophile.

Les copolymères peuvent être partiellement ou totalement salifiés.

Le mélange de monomères permettant de préparer un copolymère utilisable selon l'invention peut encore présenter les caractéristiques suivantes, prises isolément ou en combinaison :
- ledit acide organique carboxylique à insaturation éthylénique est choisi parmi l'acide acrylique et l'acide méthacrylique ;
- ledit groupement alkyle du N-alkyl acrylamide contient de 8 à 16 atomes de carbone, et en particulier de 8 à 12 atomes de carbone ;
- ledit monomère insaturé hydrophile est de préférence non ionique ; il s'agit par exemple de l'acrylamide ;
- ledit mélange de monomères contient de 30 à 60 % en poids d'acide organique carboxylique à insaturation éthylénique ;
- ledit mélange de monomères contient de 30 à 50 % en poids de N-alkyl acrylamide ;
- ledit mélange de monomères contient de O à 25 % en poids dudit monomère hydrophile.

La mesure de la masse moléculaire des copolymères n'est pas réalisable en pratique. On sait que la viscosité des solutions de polymères augmente avec la masse molaire. Pour des raisons pratiques, on utilisera, selon l'invention, de préférence les copolymères tels que définis ci-dessus, dont une solution aqueuse à 1 % en poids, contenant 5 % de laurylsulfate de sodium et 1 % de chlorure de sodium, et dans laquelle le copolymère est totalement neutralisé par l'amino-2 méthyl-2 propanol, a une viscosité au moins égale à 0,3 Pa.s (à 25°C). La viscosité est mesurée par exemple avec un viscosimètre de COUETTE. On peut utiliser notamment l'appareil commercialisé par la Société CONTRAVES sous la dénomination RHEOMAT.

Cette définition pratique de la limite inférieure préférée de la viscosité équivaut donc à celle de la limite inférieure préférée de la masse moléculaire pour les copolymères utilisables selon l'invention.

Il n'y a pas théoriquement de limite supérieure de la masse moléculaire autre que les limites inhérentes au procédé de préparation utilisé et autre que celles découlant de la nécessité de pouvoir solubiliser le copolymère au moins partiellement neutralisé dans une solution aqueuse contenant au moins 5 % de tensioactif.

Le procédé de préparation d'un copolymère tel que défini précédemment est principalement caractérisé par le fait que l'on mélange, dans les proportions requises, au moins un acide organique carboxylique à insaturation éthylénique, au moins un N-alkyl acrylamide dont le groupement N-alkyle contient de 8 à 30 atomes de carbone dans la chaîne, et éventuellement un monomère insaturé hydrophile, et que l'on soumet ledit mélange à une réaction de copolymérisation selon les méthodes connues.

Le procédé de polymérisation peut être mis en oeuvre selon une réaction de polymérisation radicalaire, en présence d'un initiateur tel que l'azo-bis-isobutyronitrile.

On peut également effectuer la préparation des copolymères de l'invention selon une réaction de polymérisation en émulsion. On citera en particulier : les procédés de polymérisation en émulsion directe ou en émulsion inverse (du type eau-dans-l'huile) ; les procédés de polymérisation "micellaire", c'est-à-dire en solution aqueuse en présence de tensioactif en quantité suffisante pour solubiliser le monomère hydrophobe (N-alkyl acrylamide) ; et les procédés de polymérisation par précipitation ou en dispersion dans un solvant organique. Ces procédés sont connus en soi. Dans les procédés de polymérisation en dispersion ou par précipitation, le solvant organique utilisé est un solvant des monomères mais un précipitant du polymère au fur et à mesure de sa formation. Ces derniers procédés sont particulièrement bien adaptés à la synthèse du copolymère de l'invention et conduisent à des poids moléculaires suffisants pour l'épaississement de milieux contenant des teneurs importantes de tensioactifs, tels que des shampooings. La viscosité des copolymères de l'invention augmentant avec leur masse moléculaire, il est en effet souhaitable d'obtenir des produits de masse moléculaire élevée afin de limiter la quantité de copolymères ajoutée dans la solution à épaissir. Par exemple, dans le cas d'un shampooing, il est préférable de n'utiliser le copolymère qu'en proportion inférieure ou égale à 5 % en poids par rapport au poids total de la composition, et de préférence inférieure ou égale à 2 % en poids.

Les copolymères de l'invention préparés par polymérisation par précipitation ou en dispersion dans un solvent organique sont obtenus sous forme de poudres faciles à décanter, à laver et à sécher.

Pour obtenir une bonne dissolution de ces copolymères dans les solutions aqueuses contenant au moins 5 % d'un tensioactif, les copolymères de l'invention sont mis en suspension dans la solution, puis neutralisés partiellement ou totalement par une base minérale ou organique. En effet, pour l'utilisation comme agent épaississant, les groupements carboxyliques présents dans les motifs dérivant du monomère acide organique carboxylique insaturé doivent être au moins partiellement salifiés, le degré de neutralisation (salification) étant suffisant pour que le polymère partiellement salifié soit soluble dans le milieu d'utilisation. En pratique, le degré de neutralisation nécessaire pour obtenir une dissolution du copolymère dans les conditions d'utilisation mentionnées ci-dessus est toujours supérieur à 50 %. Parmi les bases minérales utilisées pour la salification, on citera en particulier l'hydroxyde de sodium, l'hydroxyde de potassium et l'ammoniaque. Parmi les bases organiques utilisables pour cette salification, on citera en particulier les aminoalcools tels que l'amino-2 méthyl-2 propanol, la triéthanolamine et la triisopropanolamine.

Les copolymères de l'invention peuvent servir à préparer des compositions épaissies de solutions aqueuses de tensioactifs, constituant notamment des compositions de shampooing.

L'agent tensioactif est notamment un tensioactif anionique. On citera par exemple, à titre non limitatif, les alpha-oléfines sulfonates, ainsi que les alkylsulfates tels que le laurylsulfate de sodium, le laurylsulfate d'ammonium, le laurylsulfate de triéthanolamine et le lauryléthersulfate de sodium ; les alcanols carboxylés, les sulfosuccinates, les alkylsarcosinates et les iséthionates.

Les tensioactifs peuvent également être des tensioactifs non ioniques. On citera en particulier les alcools gras polyoxyéthylénés, les alcools gras polyglycérolés, les N-alkylamides polyglycérolés et les alkyl-éthers ou -esters de glycosides et de polyglycosides, les stérols oxyéthylénés et les phytostérols oxyéthylénés.

Les tensioactifs utilisables peuvent également être de nature amphotère : on citera en particulier les alkylbetaïnes et les alkylamidobetaïnes.

Bien entendu, la composition à épaissir pourra contenir des mélanges de tensioactifs appartenant éventuellement à des classes différentes.

L'invention a donc également pour objet l'utilisation comme agent épaississant d'un copolymère tel que défini précédemment, dans une composition aqueuse contenant au moins 5 % en poids d'un tensioactif, notamment d'un tensioactif anionique.

La composition aqueuse peut aussi contenir un électrolyte minéral.

L'invention concerne notamment l'utilisation du copolymère comme agent épaississant dans une composition aqueuse contenant le tensioactif et au moins 0,5 % d'un électrolyte minéral.

L'invention s'étend aux compositions aqueuses épaissies ainsi obtenues.

L'électrolyte minéral est par exemple un sel tel que le chlorure de sodium, le sulfate de sodium, le sulfate de magnésium, etc...

La présence de l'électrolyte minéral, de préférence le chlorure de sodium, permet d'obtenir un épaississement beaucoup plus important dans la solution de tensioactif contenant le polymère.

On a remarqué que l'électrolyte minéral ajouté, permet aussi de modifier la texture de la composition épaissie, et en particulier d'éviter un aspect filant lors de l'écoulement.

On peut également utiliser les copolymères de l'invention pour réaliser des dispersions stables de composés cosmétiques liquides non miscibles telles que des huiles et/ou des gommes de silicone, des huiles minérales ou des huiles végétales.

On peut également utiliser l'épaississement obtenu pour maintenir en suspension des substances solides utilisées dans des cosmétiques, telles que des argiles, des opacifiants ou des poudres de silicone.

En plus d'un électrolyte minéral, on peut ajouter, si on le désire, des sels organiques hydrosolubles. On peut citer par exemple le citrate de sodium, le xylènesulfonate de sodium ou d'ammonium, ou encore un filtre solaire (absorbant l'ultraviolet) comportant un motif sulfonate.

Comme indiqué ci-dessus, la composition épaissie de l'invention contient au moins un tensioactif (au moins 5 %, et généralement de 5 à 15 %, en poids) et éventuellement au moins un électrolyte minéral. La composition épaissie contient de 0,5 à 5 %, et en particulier de 0,5 à 2%, en poids de copolymère. L'électrolyte minéral est de préférence présent, dans la composition épaissie, à une concentration au moins égale à 1 % en poids, et généralement entre 1 et 2,5 % en poids.

Une telle composition constitue notamment une composition de shampooing, et peut contenir éventuellement d'autres agents cosmétiques usuels utilisés dans les shampooings.

Si désiré, le pH de la solution épaissie peut être ajusté, à l'aide d'une base convenable, généralement entre 5 et 9 et de préférence entre 5 et 7,5.

Les exemples suivants illustrent l'invention sans toutefois la limiter.

### Exemples préliminaires : préparation de produits de départ

### Synthèse du N-octyl acrylamide

Dans un réacteur contenant 0,5 mole d'octylamine, 0,5 mole de triéthylamine, 1 g d'hydroquinone monométhyl éther et 800 ml de toluène, on introduit goutte à goutte sous agitation 0,5 mole de chlorure d'acryloyle dans 200 ml de toluène. On maintient la température à 25°C par refroidissement pendant la durée de l'addition (1 heure). On laisse ensuite réagir pendant 24 heures à 25°C sous agitation. On élimine par filtration le chlorhydrate de triéthylamine formé puis on évapore le toluène sous pression réduite. Le produit obtenu est purifié par distillation sous vide avec récupération de la fraction passant entre 126 et 130°C sous 0,7 mbars. On obtient un liquide visqueux, incolore. Sa structure a été confirmée par RMN¹H.
RENDEMENT : 70 %

### Synthèse du N-dodécylacrylamide

On opère comme précédemment en remplaçant l'octylamine par la dodécylamine ; seule la purification diffère. Après filtration du chlorhydrate de triéthylamine, on lave la phase organique à l'eau. La phase toluénique est ensuite concentrée sous vide, diluée par 1 litre d'éther de pétrole, puis on effectue une cristallisation à froid. On obtient un solide blanc, cireux. Sa structure a été confirmée par RMN¹.
RENDEMENT : 85 %

### Exemple 1

On introduit successivement dans un réacteur 15,5 g d'acide acrylique, 14,2 g d'acrylamide et 20,3 g de N-dodécylacrylamide. On ajoute ensuite 300 g d'acétate de méthyle comme solvant des monomères et précipitant du polymère et 1 g d'azobisisobutyronitrile. On dissout sous agitation et barbotage d'azote le mélange de monomères en chauffant jusqu'à ébullition (57°C). Le polymère formé précipite dans le milieu sous agitation au fur et à mesure de sa formation. On laisse réagir à 57°C pendant 18 heures dans ces conditions.

Après retour à température ambiante, le polymère en suspension est filtré sous vide, lavé sur verre fritté deux fois par 300 ml d'acétate d'éthyle, récupéré et séché en étuve à 50°C sous vide jusqu'à poids constant. Le rendement obtenu après séchage est de 94 %.

### Exemples 2 à 9

Dans ces exemples, on utilise 50 g de mélange de monomères, dans des conditions de synthèse analogues à celles de l'exemple 1. Le solvant des monomères et précipitant du polymère formé est toujours l'acétate de méthyle sauf dans l'exemple 5 où on utilise le 1,2-dichloroéthane pour la polymérisation (300 g) et pour les lavages consécutifs. Dans ce dernier cas (exemple 5) on opère à 65°C. Pour maintenir un milieu suffisamment agitable pendant la polymérisation, on est obligé parfois de rajouter 300 g de solvant après une heure de réaction : c'est le cas pour les exemples 2, 4, 6 et 9. Les résultats sont rassemblés dans le tableau ci-après. La structure des polymères obtenus est confirmée par RMN¹H.

**TABLEAU 1**

| Exemple n° | Pourcentage en poids des monomères | | | | | Rendement |
|---|---|---|---|---|---|---|
| | AA | AM | A | DA | OA | |
| 2 | 42,9 | - | 18,1 | - | 39 | 95 % |
| 3 | 22,3 | 20,7 | 17,8 | 39,2 | - | 94 % |
| 4 | 38,4 | - | 16,2 | 45,4 | - | 91 % |
| 5 | 59,5 | - | - | 40,5 | - | 87 % |
| 6 | 48,8 | - | 10,7 | 40,5 | - | 93 % |
| 7 | 49,5 | - | 19,3 | 31,2 | - | 93 % |
| 8 | 41 | - | 18,4 | 40,6 | - | 91 % |
| 9 | 45,3 | - | 20,4 | - | 34,3 | 92,4 % |

| | | | | | | |
|---|---|---|---|---|---|---|
| AA : Acide acrylique | | | | | | |
| AM : Acide méthacrylique | | | | | | |
| A : Acrylamide | | | | | | |
| DA : N-dodécylacrylamide | | | | | | |
| OA : N-octylacrylamide | | | | | | |

### ETUDE DE L'EFFET EPAISSISSANT

### - a) Exemple de formulation d'un gel avec le polymère de l'exemple 1 dans du laurylsulfate de sodium éthoxylé (lauryléther sulfate de sodium)

Les proportions de réactifs sont les suivantes pour une formule sur 100 g.

Polymère n° 1 : 1 g (1 % en poids) ; amino-2 méthyl-2 propanol pour neutralisation totale des motifs carboxyliques du polymère : 383 mg ; lauryl sulfate de sodium éthoxylé : 8 g (en matière active) ; chlorure de sodium en quantité variable (voir ci-dessous) ; eau permutée q.s.p. 100 g.

La dissolution des réactifs est faite sous agitation à 70°C pendant 24 heures. Le tableau 1 ci-dessous résume les résultats de viscosités (mesurées au viscomètre Contraves à 25°C) et de texture des gels obtenus en fonction du pourcentage de chlorure de sodium dans le mélange.

**TABLEAU 1**

| % NaCl | Viscosité en Pa.s. | Texture du gel |
|---|---|---|
| 0 | 0,25 | |
| 1 | 0,49 | |
| 1,5 | 1,00 | Texture filante |
| 2 | 3,00 | Texture correcte à l'écoulement. |

On considère qu'un gel présente une texture correcte lorsqu'il peut couler, et qu'il coule sans filer. Il ne doit pas être cassant, ni élastique. Sa texture s'apparente à celle d'une crème.

Cet exemple montre l'effet du sel sur la viscosité et la texture du gel. Dans ce cas précis, on n'obtient un résultat satisfaisant qu'avec 2 % de sel.

A l'application comme shampooing capillaire, la formule contenant 2 % de chlorure de sodium comparée à une solution du tensio-actif sans le polymère, se caractérise par une meilleure formation de mousse, une meilleure répartition sur cheveux, un aspect plus crémeux et plus consistant de la mousse, une plus grande douceur, un toucher plus agréable et une plus grande légèreté sur cheveux mouillés, et sur cheveux séchés par une plus grande douceur et un toucher plus agréable, sans nuire à la légèreté.

### -b) Epaississement d'une solution de lauryl sulfate de sodium à 10 % en présence de 1 % de chlorure de sodium

Les différents polymères des exemples 1 à 9 sont comparés comme épaississants d'une même solution de tensio-actif contenant : 1 g de polymère ; amino-2 méthyl-2 propanol en quantité correspondant à la neutralisation complète du polymère ; chlorure de sodium 1 g ; lauryl sulfate de sodium 10 g ; eau permutée q.s.p. 100 g. Les résultats des mesures de viscosité (Contraves à 25°C) sont rassemblés dans le tableau 2 ci-dessous :

**TABLEAU 2**

| Polymère de l'exemple | Viscosité Pa.s. |
|---|---|
| N°1 | 3,40 |
| N°2 | 4,07 |
| N°3 | 3,02 |
| N°4 | 2,45 |
| N°5 | 1,12 |
| N°6 | 7,00 |
| N°8 | 6,00 |
| N°9 | 2,80 |

### - c) Comparaison de l'épaississement avec différents tensio-actifs

### 1°/ Tensio-actifs anioniques

On prépare des solutions aqueuses contenant un tensioactif, du chlorure de sodium, et un polymère selon l'invention.

Dans tous les cas, l'extrait sec en polymère est de 1 % en poids, et le polymère est totalement neutralisé par l'amino-2 méthyl-2 propanol.

La teneur et la viscosité des solutions étudiées sont résumées dans le tableau 3 ci-après :

**TABLEAU 3**

| Polymère de l'exemple n° | Tensio-actif | % de tensio actif | % NaCl | Viscosité (Pa.s.) |
|---|---|---|---|---|
| 1 | Lauryl sulfate de sodium | 10 | 0 | 0,44 |
| | Lauryl sulfate de sodium | 10 | 1 | 3,40 |
| | Lauryl éther sulfate de sodium | 8 | 0 | 0,25 |
| | Lauryl éther sulfate de sodium | 8 | 1 | 0,49 |
| | Lauryl éther sulfate de sodium | 8 | 2 | 3,00 |
| | Lauryl sulfate de triéthanolamine | 10 | 0 | 0,45 |
| | Lauryl sulfate de triéthanolamine | 10 | 1 | 0,95 |
| | Lauryl sulfate de triéthanolamine | 10 | 2 | ≥ 3,00 |
| 8 | Lauryl sulfate de sodium | 10 | 0 | 0,35 |
| | Lauryl sulfate de sodium | 10 | 1 | 6,00 |
| | Lauryl éther sulfate de sodium | 8 | 0 | 0,13 |
| | Lauryl éther sulfate de sodium | 8 | 1 | 0,40 |
| | Lauryl sulfate de triéthanolamine | 10 | 0 | 0,31 |
| | Lauryl sulfate de triéthanolamine | 10 | 1 | 1,20 |
| | Lauryl sulfate de triéthanolamine | 10 | 2 | 3,00 |

Le lauryl éther sulfate de sodium, oxyéthyléné à 2,2 moles d'oxyde d'éthylène, est celui commercialisé par la Société HENKEL sous la dénomination SIPON A05-225.

### 2°/ Tensio-actif amphotère

Le tensioactif est la cocodiméthylsulfopropylbetaïne décrite dans le Dictionnaire CTFA sous le nom de "coco-sultaïne". La concentration du polymère est de 1,5 % et les fonctions acides sont totalement neutralisées par l'amino-2 méthyl-2 propanol. Le tensioactif est utilisé à une concentration de 10 %. Les résultats sont les suivants (Tableau 4).

**TABLEAU 4**

| Polymère de l'exemple N° | % NaCl | Viscosité en Pa.s. |
|---|---|---|
| 8 | 1 | 2,0 |
| 8 | 2 | 1,4 |

### 3°/ Tensioactif non ionique

Le tensioactif est le 1,2-dodécanediol éthérifié par 3,5 motifs de glycérol décrit dans le brevet français 2.091.516 (71.17206). La concentration en polymère est de 1,5 %. Le polymère est neutralisé totalement par l'amino-2 méthyl-2 propanol. La concentration du tensioactif est de 10 %. Les résultats sont les suivants (Tableau 5) :

**TABLEAU 5**

| Polymère de l'exemple N° | % NaCl | Viscosité en Pa.s. |
|---|---|---|
| 2 | 1 | 1,40 |
| 2 | 2 | 1,25 |
| 8 | 1 | 0,28 |
| 8 | 2 | 7,00 |

## Revendications

1. Utilisation comme agent épaississant d'un copolymère résultant de la copolymérisation d'un mélange de monomères comprenant :
- au moins 30 % en poids d'au moins un acide organique carboxylique à insaturation éthylénique ;
- au moins 30 % en poids d'au moins un N-alkyl acrylamide dont le groupement alkyle contient de 8 à 30 atomes de carbone dans la chaîne alkyle ;
- et de 0 à 40 % d'au moins un monomère insaturé hydrophile ; ainsi qu'un copolymère correspondant dont les groupements carboxyliques sont totalement ou partiellement salifiés.

2. Utilisation selon la revendication 1, caractérisée par le fait que ledit acide organique carboxylique à insaturation éthylénique est choisi parmi l'acide acrylique et l'acide méthacrylique.

3. Utilisation selon l'une quelconque des revendications précédentes, caractérisée par le fait que ledit groupement alkyle du N-alkyl acrylamide contient de 8 à 16 atomes de carbone, et en particulier 8 à 12 atomes de carbone.

4. Utilisation selon l'une quelconque des revendications précédentes, caractérisée par le fait que ledit monomère insaturé hydrophile est non ionique.

5. Utilisation selon l'une quelconque des revendications précédentes, caractérisée par le fait que ledit monomère insaturé hydrophile est l'acrylamide.

6. Utilisation selon l'une quelconque des revendications précédentes, caractérisée par le fait que ledit mélange de monomères contient de 30 à 60 % en poids d'acide organique carboxylique à insaturation éthylénique.

7. Utilisation selon l'une quelconque des revendications précédentes, caractérisée par le fait que ledit mélange de monomères contient de 30 à 50 % en poids de N-alkyl acrylamide.

8. Utilisation selon l'une quelconque des revendications précédentes, caractérisée par le fait que ledit mélange de monomères contient de 0 à 25 % en poids dudit monomère insaturé hydrophile.

9. Utilisation selon l'une quelconque des revendications précédentes, caractérisée par le fait que les groupements carboxyliques que le copolymère contient sont partiellement ou totalement salifiés.

10. Utilisation selon l'une quelconque des revendications précédentes, caractérisée par le fait qu'au moins 50 % des groupements carboxyliques que le copolymère contient sont salifiés.

11. Utilisation selon l'une quelconque des revendications précédentes, caractérisée par le fait qu'une solution aqueuse à 1 % en poids dudit copolymère, contenant 5 % de laurylsulfate de sodium et 1 % de chlorure de sodium, et dans laquelle le copolymère est totalement neutralisé par l'amino-2 méthyl-2 propanol, a une viscosité au moins égale à 0,3 Pa.s (à 25°C).

12. Utilisation comme agent épaississant pour une composition aqueuse, d'un copolymère tel que défini dans l'une quelconque des revendications 1 à 11, ladite composition aqueuse contenant au moins 5 % en poids d'au moins un tensioactif, ledit copolymère étant au moins partiellement salifié pour être solubilisé dans ladite composition.

13. Utilisation selon la revendication 12, caractérisée par le fait que ledit copolymère est salifié par l'hydroxyde de sodium ou de potassium, par l'ammoniaque ou par une base organique.

14. Utilisation selon la revendication 13, caractérisée par le fait que ladite base organique est un aminoalcool.

15. Utilisation selon l'une quelconque des revendications 12 à 14, caractérisée par le fait que ladite composition aqueuse contient en outre au moins un électrolyte minéral.

16. Utilisation selon la revendication 15, caractérisée par le fait que ladite composition aqueuse contient au moins 0,5 % en poids dudit électrolyte minéral.

17. Utilisation selon l'une quelconque des revendications 15 et 16, caractérisée par le fait que ledit électrolyte minéral est un sel.

18. Utilisation selon la revendication 17, caractérisée par le fait que ledit sel est choisi parmi le chlorure de sodium, le sulfate de sodium et le sulfate de magnésium.

19. Utilisation selon l'une quelconque des revendications 15 à 18, caractérisée par le fait que ledit électrolyte minéral est présent dans la composition à une concentration au moins égale à 1 % en poids.

20. Utilisation selon l'une quelconque des revendications 1 à 19, caractérisée par le fait que ledit copolymère est présent à raison de 0,5 à 5%, et en particulier de 0,5 à 2 % en poids par rapport au poids total de la composition.

21. Utilisation selon l'une quelconque des revendications 12 à 20, caractérisée par le fait que ladite composition est une composition de shampooing.

22. Composition aqueuse, caractérisée par le fait qu'elle contient comme agent épaississant, sous forme au moins partiellement salifiée, au moins un copolymère tel que défini dans l'une quelconque des revendications 1 à 11, et que ladite composition contient au moins 5 % d'au moins un tensioactif.

23. Composition selon la revendication 22, caractérisée par le fait que ledit tensioactif est au moins un tensioactif anionique.

24. Composition selon l'une quelconque des revendications 22 et 23, caractérisée par le fait qu'elle contient de 0,5 à 5 % et en particulier de 0,5 à 2% en poids dudit copolymère.

25. Composition selon l'une quelconque des revendications 22 à 24, caractérisée par le fait qu'elle contient en outre au moins un électrolyte minéral.

26. Composition selon la revendication 25, caractérisée par le fait qu'elle contient au moins 0,5 % en poids dudit électrolyte minéral.

27. Composition selon l'une quelconque des revendications 25 et 26, caractérisée par le fait que ledit électrolyte minéral est un sel.

28. Composition selon la revendication 27, caractérisée par le fait que ledit électrolyte minéral est choisi parmi le chlorure de sodium, le sulfate de sodium et le sulfate de magnésium.

29. Composition selon la revendication 27 ou 28, caractérisée par le fait qu'elle contient au moins 1 % en poids dudit sel.

## Patentansprüche

1. Verwendung eines Copolymeren als Verdickungsmittel, das aus der Copolymerisation eines Monomerengemisches hervorgeht, das enthält:
- mindestens 30 Gew.-% einer ethylenisch ungesättigten organischen Carbonsäure,
- mindestens 30 Gew.-% eines N-Alkylacrylamids, dessen Alkylgruppe 8 bis 30 Kohlenstoffatome in der Alkylkette enthält, und
- 0 bis 40 Gew.-% eines hydrophilen ungesättigten Monomeren,
sowie ein entsprechendes Copolymeren mit teilweise oder vollständig in Form von Salzen vorliegenden Carbonsäuregruppen.

2. Verwendung gemäß Anspruch 1, dadurch gekennzeichnet, daß die ethylenisch ungesättigte organische Carbonsäure unter Acrylsäure und Methacrylsäure ausgewählt ist.

3. Verwendung gemäß einem der vorstehenden Ansprüche, dadurch gekennzeichnet, daß die Alkylgruppe des N-Alkylacrylamids 8 bis 16, vorzugsweise 8 bis 12 Kohlenstoffatome enthält.

4. Verwendung gemäß einem der vorstehenden Ansprüche, dadurch gekennzeichnet, daß das hydrophile ungesättigte Monomer ein nichtionisches Monomer ist.

5. Verwendung gemäß einem der vorstehenden Ansprüche, dadurch gekennzeichnet, daß das hydrophile ungesättigte Monomer Acrylamid ist.

6. Verwendung gemäß einem der vorstehenden Ansprüche, dadurch gekennzeichnet, daß das Monomerengemisch 30 bis 60 Gew.-% der ethylenisch ungesättigten organischen Carbonsäure enthält.

7. Verwendung gemäß einem der vorstehenden Ansprüche, dadurch gekennzeichnet, daß das Monomerengemisch 30 bis 50 Gew.-% N-Alkylacrylamid enthält.

8. Verwendung gemäß einem der vorstehenden Ansprüche, dadurch gekennzeichnet, daß das Monomerengemisch 0 bis 25 Gew.-% des hydrophilen ungesättigten Monomeren enthält.

9. Verwendung gemäß einem der vorstehenden Ansprüche, dadurch gekennzeichnet, daß die enthaltenen Carbonsäuregruppen teilweise oder vollständig versalzt sind.

10. Verwendung gemäß einem der vorstehenden Ansprüche, dadurch gekennzeichnet, daß mindestens 50 % der enthaltenen Carbonsäuregruppen versalzt sind.

11. Verwendung gemäß einem der vorstehenden Ansprüche, dadurch gekennzeichnet, daß eine wäßrige Lösung mit 1 Gew.-% des Copolymeren, die 5 % Natriumlaurylsulfat und 1 % Natriumchlorid enthält und in der das Copolymer vollständig mit 1-Amino-2-methyl-2-propanol neutralisiert ist, eine Viskosität von mindestens 0,3 Pa • s (bei 25 °C) aufweist.

12. Verwendung als Verdickungsmittel einer wäßrigen Zubereitung eines Copolymeren gemäß einem der Ansprüche 1 bis 11, wobei die wäßrige Zubereitung mindestens 5 Gew.-% mindestens eines Tensids enthält und das Copolymer mindestens teilweise als Salz vorliegt, um in der Zubereitung löslich zu sein.

13. Verwendung gemäß Anspruch 12, dadurch gekennzeichnet, daß das Copolymer mit Natrium- oder Kaliumhydroxid, Ammoniak oder einer organischen Base in ein Salz überführt wird.

14. Verwendung gemäß Anspruch 13, dadurch gekennzeichnet, daß die organische Base ein Aminoalkohol ist.

15. Verwendung gemäß einem der Ansprüche 12 - 14, dadurch gekennzeichnet, daß die wäßrige Zubereitung zusätzlich mindestens einen mineralischer Elektrolyten enthält.

16. Verwendung gemäß Anspruch 15, dadurch gekennzeichnet, daß die wäßrige Zubereitung mindestens 0,5 Gew.-% an mineralischem Elektrolyt enthält.

17. Verwendung gemäß einem der Ansprüche 15 und 16, dadurch gekennzeichnet, daß es sich bei dem mineralischer Elektrolyten um ein Salz handelt.

18. Verwendung gemäß Anspruch 17, dadurch gekennzeichnet, daß das Salz unter Natriumchlorid, Natriumsulfat und Magnesiumsulfat ausgewählt ist.

19. Verwendung gemäß einem der Ansprüche 15 bis 18, dadurch gekennzeichnet, daß der mineralische Elektrolyt in der Zubereitung in einer Konzentration von mindestens 1 Gew.-% vorhanden ist.

20. Verwendung gemäß einem der Ansprüche 1 bis 19, dadurch gekennzeichnet, daß das Copolymer in einer Menge von 0,5 bis 5 %, insbesondere 0,5 bis 2 Gew.-%, bezogen auf das Gesamtgewicht der Zubereitung, vorhanden ist.

21. Verwendung gemäß einem der Ansprüche 12 bis 20, dadurch gekennzeichnet, daß es sich bei der Zubereitung um ein Shampoo handelt.

22. Wäßrige Zubereitung, dadurch gekennzeichnet, daß sie als Verdickungsmittel in mindestens teilweise in ein Salz überführter Form mindestens ein Copolymer gemäß einem der Ansprüche 1 bis 11 enthält und daß die Zubereitung mindestens 5 % mindestens eines Tensids enthält.

23. Zubereitung gemäß Anspruch 22, dadurch gekennzeichnet, daß das Tensid mindestens ein anionisches Tensid ist.

24. Zubereitung gemäß einem der Ansprüche 22 und 23, dadurch gekennzeichnet, daß sie 0,5 bis 5 %, insbesondere 0,5 bis 2 Gew.-% des Copolymeren enthält.

25. Zubereitung gemäß einem der Ansprüche 22 bis 24, dadurch gekennzeichnet, daß sie zusätzlich mindestens einen mineralischen Elektrolyten enthält.

26. Zubereitung nach Anspruch 25, dadurch gekennzeichnet, daß sie mindestens 0,5 Gew.- % mineralischen Elektrolyten enthält.

27. Zubereitung gemäß einem der Ansprüche 25 und 26, dadurch gekennzeichnet, daß es sich bei dem mineralischen Elektrolyten um ein Salz handelt.

28. Zubereitung gemäß Anspruch 27, dadurch gekennzeichnet, daß der mineralische Elektrolyt unter Natriumchlorid, Natriumsulfat und Magensiumsulfat ausgewählt ist.

29. Zubereitung gemäß Anspruch 28 und 29, dadurch gekennzeichnet, daß sie mindestens 1 Gew.-% des Salzes enthält.

## Claims

1. Use as thickening agent of a copolymer resulting from the copolymerization of a mixture of monomers comprising:
- at least 30% by weight of at least one organic carboxylic acid containing ethylenic unsaturation;
- at least 30% by weight of at least one N-alkylacrylamide in which the alkyl group contains from 8 to 30 carbon atoms in the alkyl chain;
- and from 0 to 40% of at least one hydrophilic unsaturated monomer;
and a corresponding copolymer in which the carboxyl groups are completely or partially salified.

2. Use according to Claim 1, characterized in that the said organic carboxylic acid containing ethylenic unsaturation is chosen from acrylic acid and methacrylic acid.

3. Use according to either one of the preceding claims, characterized in that the said alkyl group of the N-alkylacrylamide contains from 8 to 16 carbon atoms and in particular 8 to 12 carbon atoms.

4. Use according to any one of the preceding claims, characterized in that the said hydrophilic unsaturated monomer is non-ionic.

5. Use according to any one of the preceding claims, characterized in that the said hydrophilic unsaturated monomer is acrylamide.

6. Use according to any one of the preceding claims, characterized in that the said mixture of monomers contains from 30 to 60% by weight of organic carboxylic acid containing ethylenic unsaturation.

7. Use according to any one of the preceding claims, characterized in that the said mixture of monomers contains from 30 to 50% by weight of N-alkylacrylamide.

8. Use according to any one of the preceding claims, characterized in that the said mixture of monomers contains from 0 to 25% by weight of the said hydrophilic unsaturated monomer.

9. Use according to any one of the preceding claims, characterized in that the carboxyl groups which the copolymer contains are partially or completely salified.

10. Use according to any one of the preceding claims, characterized in that at least 50% of the carboxyl groups which the copolymer contains are salified.

11. Use according to any one of the preceding claims, characterized in that a 1% by weight aqueous solution of the said copolymer, containing 5% of sodium lauryl sulphate and 1% of sodium chloride, in which the copolymer is completely neutralized with 2-amino-2-methylpropanol, has a viscosity of at least 0.3 Pa·s (at 25°C).

12. Use as thickening agent for an aqueous composition of a copolymer as defined in any one of Claims 1 to 11, the said aqueous composition containing at least 5% by weight of at least one surfactant, the said copolymer being at least partially salified in order to be dissolved in the said composition.

13. Use according to Claim 12, characterized in that the said copolymer is salified with sodium hydroxide or potassium hydroxide, with ammonia or with an organic base.

14. Use according to Claim 13, characterized in that the said organic base is an aminoalcohol.

15. Use according to any one of Claims 12 to 14, characterized in that the said aqueous composition additionally contains at least one inorganic electrolyte.

16. Use according to Claim 15, characterized in that the said aqueous composition contains at least 0.5% by weight of the said inorganic electrolyte.

17. Use according to either one of Claims 15 and 16, characterized in that the said inorganic electrolyte is a salt.

18. Use according to Claim 17, characterized in that the said salt is chosen from sodium chloride, sodium sulphate and magnesium sulphate.

19. Use according to any one of Claims 15 to 18, characterized in that the said inorganic electrolyte is present in the composition at a concentration of at least 1% by weight.

20. Use according to any one of Claims 1 to 19, characterized in that the said copolymer is present in the proportion of 0.5 to 5% and in particular of 0.5 to 2% by weight with respect to the total weight of the composition.

21. Use according to any one of Claims 12 to 20, characterized in that the said composition is a shampoo composition.

22. Aqueous composition, characterized in that it contains as thickening agent, in an at least partially salified form, at least one copolymer as defined in any one of Claims 1 to 11 and in that the said composition contains at least 5% of at least one surfactant.

23. Composition according to Claim 22, characterized in that the said surfactant is at least an anionic surfactant.

24. Composition according to either one of Claims 22 and 23, characterized in that it contains from 0.5 to 5% and in particular from 0.5 to 2% by weight of the said copolymer.

25. Composition according to any one of Claims 22 to 24, characterized in that it additionally contains at least one inorganic electrolyte.

26. Composition according to Claim 25, characterized in that it contains at least 0.5% by weight of the said inorganic electrolyte.

27. Composition according to either one of Claims 25 and 26, characterized in that the said inorganic electrolyte is a salt.

28. Composition according to Claim 27, characterized in that the said inorganic electrolyte is chosen from sodium chloride, sodium sulphate and magnesium sulphate.

29. Composition according to Claim 27 or 28, characterized in that it contains at least 1% by weight of the said salt.
